(19) Europäisches Patentamt
European Patent Office
Office européen des brevets

(11) **EP 3 402 446 B1**

(12) **EUROPEAN PATENT SPECIFICATION**

(45) Date of publication and mention of the grant of the patent:
**24.03.2021 Bulletin 2021/12**

(51) Int Cl.:
*A61F 2/90* *(2013.01)* *A61F 2/24* *(2006.01)*
*A61F 2/07* *(2013.01)*

(21) Application number: **17700804.2**

(86) International application number:
**PCT/EP2017/050568**

(22) Date of filing: **12.01.2017**

(87) International publication number:
**WO 2017/121803 (20.07.2017 Gazette 2017/29)**

(54) **IMPLANTABLE PROSTHESIS FOR THORACIC AORTIC DISEASE INVOLVING AORTIC VALVE DYSFUNCTION**

IMPLANTIERBARE PROTHESE FÜR THORAKALE AORTENERKRANKUNG MIT AORTENKLAPPENDYSFUNKTION

PROTHÈSE IMPLANTABLE POUR UNE MALADIE DE L'AORTE THORACIQUE IMPLIQUANT UNE DYSFONCTION DE VALVULE AORTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.01.2016 EP 16151194**

(43) Date of publication of application:
**21.11.2018 Bulletin 2018/47**

(73) Proprietor: **Cardiatis S.A.**
**5032 Isnes (BE)**

(72) Inventors:
• **FRID, Noureddine**
**1650 Beersel (BE)**

• **BRONSON DIETHRICH, Edward**
**Phoenix**
**Arizona 85253 (US)**

(74) Representative: **Pronovem**
**Office Van Malderen**
**Avenue Josse Goffin 158**
**1082 Bruxelles (BE)**

(56) References cited:
**US-A1- 2009 125 098    US-A1- 2012 116 498**
**US-A1- 2013 144 373**

## Description

## Field of the invention

The present invention relates to implantable endoluminal prostheses. More particularly, it relates to an endoluminal prosthesis for treatment of a thoracic aortic disease, such as aneurysm and dissection of the root and/or ascending aorta. Even more particularly it relates to an endoluminal prosthesis for treatment of a thoracic aortic disease involving cardiac valve dysfunction such as aortic valve regurgitation or aortic valve stenosis.

## Background of the invention

Thoracic aneurysms and dissections involve one or more aortic segments such as aortic root, ascending aorta, arch and descending aorta, and are classified accordingly. Sixty percent of thoracic aortic aneurysms involve the aortic root and/or ascending aorta, 40% involve the descending aorta, 10% involve the arch, and 10% involve the thoracoabdominal aorta.

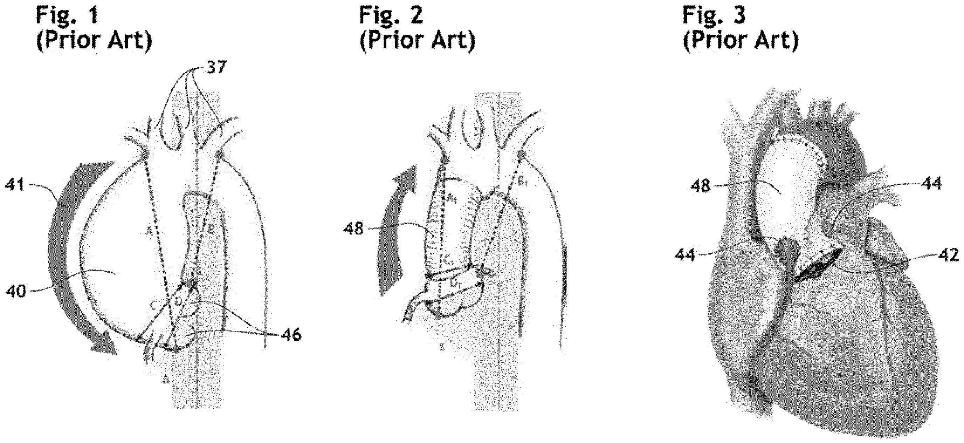

Dilatation of the ascending aorta (i.e., ascending aortic aneurysm 40) illustrated in FIG.1 is known as a common cause of aortic regurgitation because the ascending aneurysm grows not only in diameter but also in length. Such elongation may cause aortic valve 46 incompetence by dislocation of the aortic valve plane towards the left ventricle (arrow 41) and subsequent valve dislocation, causing leaflet prolapse.

Treatment of ascending aortic aneurysm 40 usually requires open surgical repair implying cardiopulmonary bypass (there is no "off-the-pump" option), and generally resecting the aneurysm 40 and replacing the vessel with a prosthetic Dacron tube graft 48 of appropriate size as shown in FIG.2.

When the aneurysm 40 involves the aortic root and is associated with significant aortic regurgitation, one usually performs a composite aortic repair (Bentall procedure) by using a tube graft 48 with a prosthetic aortic valve sewn to one end. The valve and graft are sewn directly to the aortic annulus 42 and the coronary arteries 44 are then reimplanted into the Dacron aortic graft 48 as illustrated in FIG.3. Endovascular repair is also known as a relatively new and minimally invasive technique for treatment of abdominal aortic aneurysm. It delivers an impermeable tube (graft) supported with metallic or plastic frame (stent) via a remote vessel. However, because of its impermeability, this technique cannot be applied to ascending aneurysm repair in which the aneurysm involves important branches (e.g. the coronary arteries 44 and the supra aortic branches 37), otherwise it causes fatal complications with occlusion of the branches.

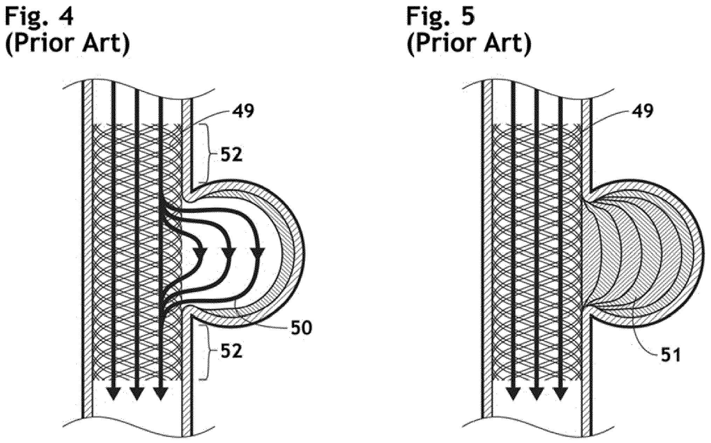

A new type of aneurysm repair system with a multilayer braided stent (MBS, 49) described in US Pat. No. 7588597 and 8192484 was recently introduced by Frid et al. The repair system comprises a bare (i.e. devoid of any impermeable cover layer) self-expandable metal stent 49 in a straight configuration. MBS consists of a plurality of interconnected layers (i.e. multilayer structure) formed by braiding a plurality of wires. A lattice is defined by the interconnected layers and provides the MBS with an optimized porosity. Instead of mechanically/physically keeping out the blood flow from the aneurysm, MBS allows the blood to flow into the aneurysm sac through its multilayer structure, converting an undesired damaging turbulence in the aneurysmal sac into a smooth laminar flow 50 (FIG.4), which results in excluding the aneurysm by forming a protecting organized thrombus 51 known as layers of Zhan (FIG.5), while keeping the branches and collaterals patent.

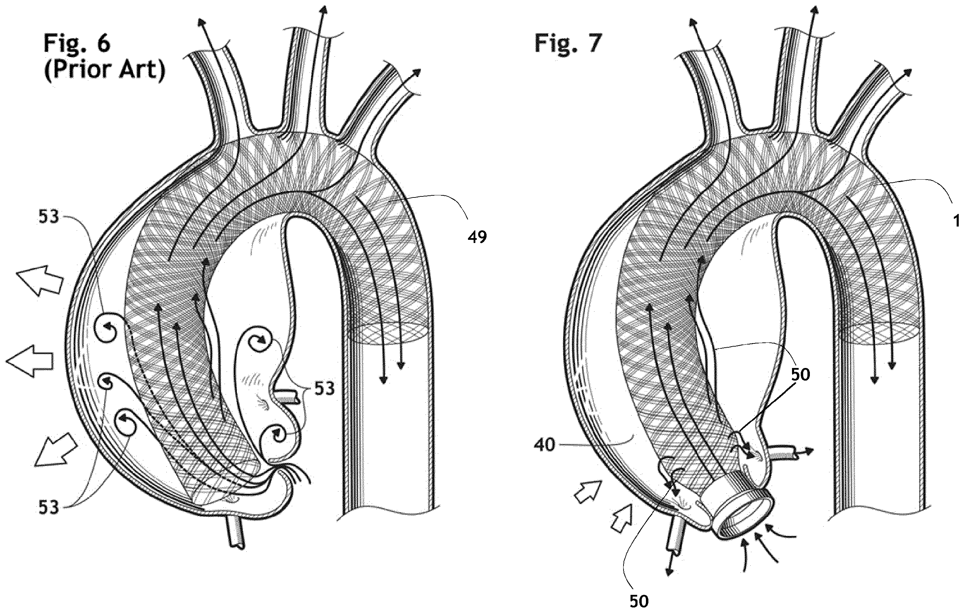

However, a conventional straight multilayer braided stent (MBS) is not suitable to treat the ascending aneurysm 40 because no adequate healthy landing zones 52 for MBS implantation are available. In order to make the protecting organize thrombus 51, the blood flow in the aneurysmal sac should be laminated. If an adequate healthy landing zone 52 at the beginning of the MBS is missing, a gap may occur between the aortic wall and the MBS 49. This lack of sealing allows undesired turbulence 53 formation in the aneurysmal sac, a phenomenon which is called endoleak, resulting in enlargement of the aneurysm with localized stress brought by turbulence 53 as shown in FIG.6

In US2013144373 a device and method for endovascular repair of a patient's aorta is disclosed. The device includes a frame component that has a balloon-expandable frame and a self-expanding frame secured to the balloon-expandable frame. The device also includes a valve element positioned at the proximal end of the frame component.

## Summary of the invention

A first object of the present invention is to provide a device implantable by endovascular approach for treatment of ascending aortic aneurysm.

Another object of the present invention is to provide a device implantable by endovascular approach for treatment of a valve dysfunction involving ascending aortic aneurysm.

Another object of the invention is ensuring a sealing at a proximal end of a cardiac device in order to reduce the risk of aneurysm rupture.

Another object of the invention is ensuring patency of the coronary arteries while treating an ascending aortic aneurysm or a heart valve dysfunction.

Another object of the invention is ensuring a firm support for an artificial heart valve.

It is still another object of the present invention to provide an implantable medical device and a method for improving the perfusion of organs by lamination through the device, such as the heart through coronaries and brain through the brachiocephalic trunk.

The subject of the present invention is defined in the appended independent claims. Preferred embodiment are defined in the depended claims.

A subject of the present invention is an implantable endoluminal prosthesis suitable for deployment from the aortic annulus to the aorta. The prosthesis comprises a self-expandable braided framework able to expand from a radially compressed state in a delivery configuration to a radially expanded state. The self-expandable braided framework is formed of braided wires having a given diameter ($\varnothing_{25}$) and having a proximal end configured to extend toward the heart and a distal end configured to extent toward away from the heart. The self-expandable braided framework extends along an axis. The self-expandable braided framework comprises a main tubular body comprising a lumen in a cylindrical form with a circular cross-section and a constant diameter at the distal end of the self-expandable braided framework, a neck comprising a lumen in a cylindrical form with a circular cross-section and a constant diameter smaller than the one of said main tubular body at the proximal end of the self-expandable braided framework, and a transition portion extending between the proximal end of the main tubular body and the distal end of the neck. The main tubular body, said neck and said transition portion consists of an integrated structure being devoid of any impermeable cover layer, and forming a wall having a thickness ($T_{20}$). The prosthesis further comprises a radially collapsible valve body comprising an impermeable material placed within the lumen of the neck. In the fully expanded state, the total length of the main tubular body and the transition portion is at least 50 mm, preferably at least 100 mm, more preferably at least 150 mm, even more preferably at least 200 mm. The self-expandable braided framework comprises a plurality of layers of wires made of biocompatible material, Each layer forming a mesh, the meshes forming a lattice with a plurality of wires of given layers. The lattice, when observed normal to a wall of the self-expandable braided framework, defines polygonal opening units. Said biocompatible material is preferably selected from the group consisting of titanium, nickel-titanium alloys such as nitinol and Nitinol-DFT®-Platinum, any type of stainless steels, or a cobalt-chromium-nickel alloys such as Phynox®.

According to the invention, a ratio ($T_{20}/\varnothing_{25}$) of the thickness ($T_{20}$) of a wall of the self-expandable braided framework to the diameter ($\varnothing_{25}$) of wire is higher than 2.0, preferably at least 3.5, more preferably at least 5.5, even more preferably at least 6.5, still even more preferably at least 7.5.

The self-expandable braided framework comprises less than 150 wires, preferably at least 90 wires and at most 130 wires. Advantageously, the diameter of wire is more than 180 $\mu$m, preferably at least 200 $\mu$m and at most 220 $\mu$m.

Advantageously, the meshes are interlocked forming a lattice with a plurality of wires of given layers, the wires being interlocked in the mesh of at least one of the adjacent layers.

In a fully expanded state, a surface coverage ratio (SCR) of said self-expandable braided framework is preferably at least 25% and at most 50%, preferably at least 30% and at most 40%, more preferably at most 35%.

According to a preferable embodiment, the self-expandable braided framework further comprises a sealing portion between the proximal end of the braided frame work and the neck, the diameter of sealing portion increasing toward the proximal end of the braided framework.

According to another preferable embodiment, the self-expandable braided framework further comprises an enlarged portion between the distal end of the self-expandable braided framework and the main tubular body, the diameter of enlarged portion increasing toward the distal end of the self-expandable braided framework.

Another subject of the present invention relates to the implantable prosthesis described above for use in treatment for cardiac valve dysfunction involving ascending aortic aneurysm, such as aortic valve regurgitation and aortic valve stenosis.

Another subject of the present invention relates to the implantable prosthesis described above for use in improving perfusion of an organ by covering with said implantable endoluminal prosthesis orifices of the coronaries and the supra aortic branches which carries blood to the heart and the brain.

**Brief Description of the Figures**

Other particularities and advantages of the invention will be developed hereinafter, reference being made to the appended drawings wherein:

FIG.1 is a sketch view of an ascending aortic aneurysm involving cardiac valve dysfunction;
FIGs.2 and 3 are respectively a sketch view and a view in perspective of an ascending aorta partially replaced with artificial graft by open surgical repair;
FIG.4 is a schematic longitudinal cut view of a laminated blood flow formed in an aneurysm after implantation of a multilayer braided stent;
FIG.5 is a schematic longitudinal cut view of an organized thrombus formed in an aneurysm after implantation of a conventional straight multilayer braided stent (MBS);
FIG.6 is a partially cutaway elevation view of an ascending aortic aneurysm involving cardiac valve dysfunction and conventional straight MBS deployed therein;
FIG.7 is a side view of an implantable endoluminal prosthesis according to the invention placed in the ventricle of the heart and in the ascending aorta, the arch and the descending aorta;
FIG.8a is a side view of the prosthesis of FIG.7 in fully expanded state;
FIGs.8b and 8c are bottom views of the device of FIG.8a, respectively with closed an open heart valve;
FIG.9 is a side view of another embodiment of the prosthesis of the invention in fully expanded state;
FIGs.10a and 10b are perspective views of the tissues forming the valve body;
FIGs.11 and 12 are side views of other embodiments of the prosthesis of the invention in fully expanded state;

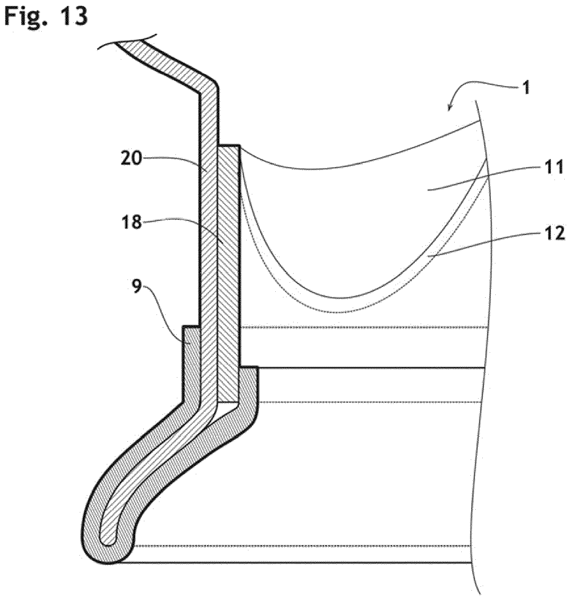

FIG.13 is a cut view of a detail of another embodiment of the prosthesis of the invention;
FIG.14 is a cut view of another embodiment of the prosthesis of the invention placed in the ventricle of the heart and in the ascending aorta;
FIG.15 is a top view of a prosthesis according to the present invention in expanded state;
FIG.15a is a schematic magnified view of a portion of the endoluminal prosthesis illustrated in FIG.15.
FIG.16 is a side view of a tubular body deployed in a curved lumen;
FIGs.17 and 18 are perspective views of the device of the invention, respectively in straight fully expanded state and in deployed state in a curved lumen;
FIG.19 is a schematic magnified view of a portion of a wall of an endoluminal prosthesis according to the present invention.

**Detailed Description of the Invention**

As used hereinafter, the term "implantable" refers to an ability of a medical device to be positioned at a location within a body vessel. Implantable medical device can be configured for transient placement within a body vessel during a medical intervention (e.g., seconds, minutes, hours), or to remain in a body vessel permanently.

The terms "endoluminal" or "transluminal" prosthesis refers to a device adapted for placement in a curved or straight body vessel by procedures wherein the prosthesis is advanced within and through the lumen of a body vessel from a remote location to a target site within the body vessel. In vascular procedures, a medical device can typically be introduced "endovascularly" using a catheter over a wire guide under fluoroscopic guidance. The catheters and wire guides may be introduced through conventional access sites in the vascular system.

The term "catheter" refers to a tube that is inserted into a blood vessel to access the target site. In the present description, a "catheter" will designate either a catheter per se, or a catheter with its accessories, meaning needle, guide wire, introducer sheath and other common suitable medical devices known by the man skilled in the art.

The term "endothelialisation" refers to a cellular process resulting in ingrowth of endothelial cells onto a device.

The term "permanent" refers to a medical device which may be placed in a blood vessel and will remain in the blood vessel for a long period of time (e.g. months, years) and possibly for the remainder of the patient's life.

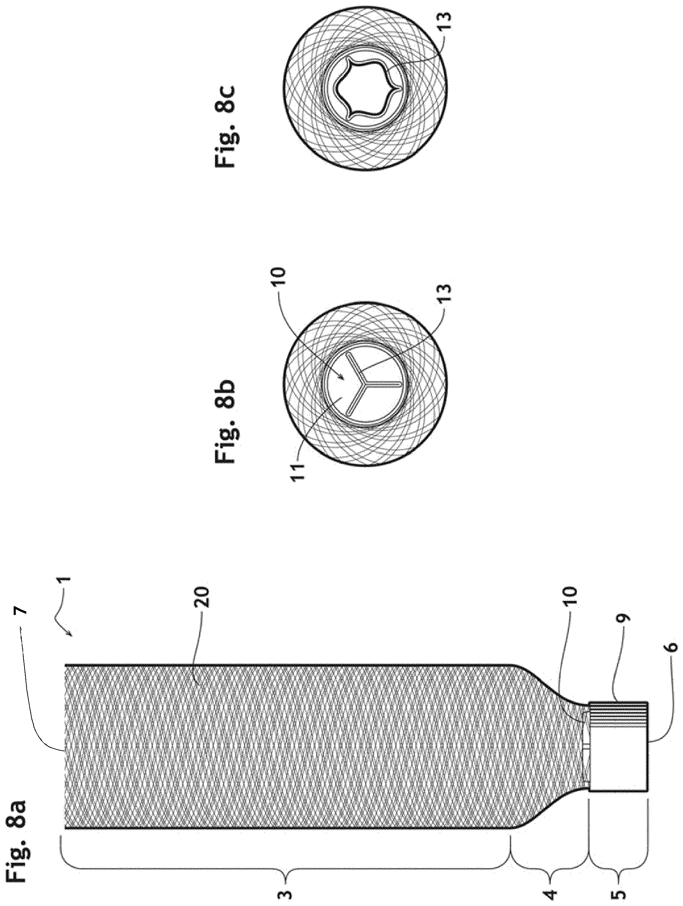

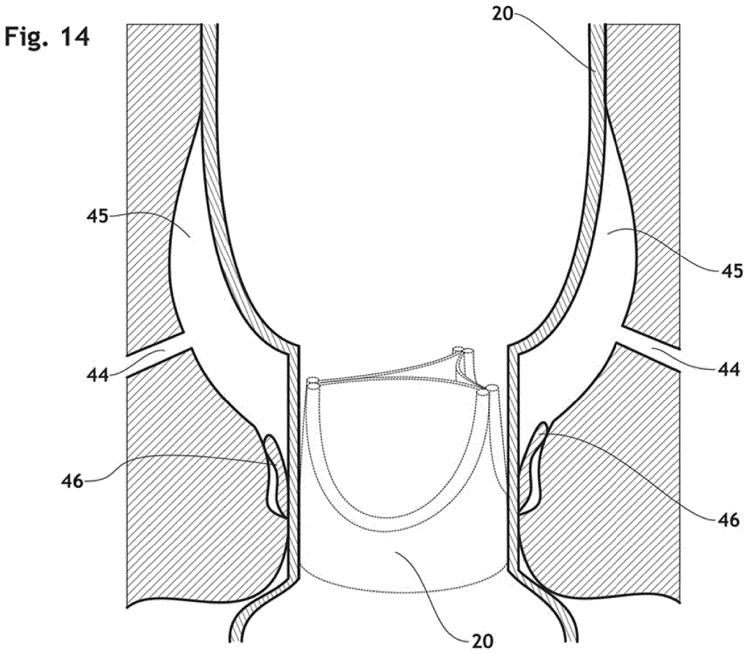

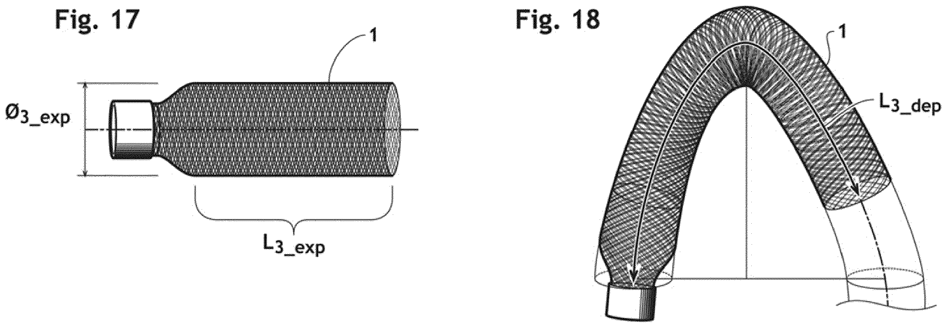

The endoluminal prosthesis **1** is configured to take a compressed shape having a relatively small and relatively uniform diameter when disposed within a delivery system (i.e., "in compressed state"), and to spontaneously take a deployed shape with radially expanded diameter within the delivery location such as a body lumen (i.e., "in deployed state") as shown in FIGs. 7 and 14. As used herein the terms "expanded shape" or "expanded state" refer to a shape or state resulting from the self-expanding properties of a self-spring-back object (e.g., braided framework **20**) when it is allowed to expand without any outer compression force (i.e., non-constricted state) as for example shown in FIGs. 8a to 8c, 9, 5, 11 and 12. Beside these definitions, the term "nominal diameter" designates the diameter of the implantable endoluminal prosthesis when placed in the targeted vessel. Generally, the nominal diameter ($\varnothing_{nor}$) of a self-expandable device designed to be placed permanently inside a body lumen is 10 to 25% smaller than the external diameter of said device when deployed without external compression force ($\varnothing_{exp}$). Since the diameter ($\varnothing_{39}$) of the aorta is generally between 20 mm and 40 mm, the main tubular body **3** of the self-expandable braided framework **20** is accordingly designed and/or manufactured to have a diameter ($\varnothing_{3\_exp}$) between 22 mm and 50 mm in expanded state. Variations of the diameter of the prosthesis influence, in turn, its length. As shown in FIGs. 17 and 18, the length ($L_{3\_dep}$) of the main tubular body **3** of the invention in deployed state is thus larger than its length ($L_{3\_exp}$) in expanded state. The length-related compression ratio (**LCR**) of the main tubular body 3 can be defined by the relation:

$$LCR = \left(L_{3\_dep} - L_{3\_exp}\right)/L_{3\_exp}$$

FIG. 7 represents an implantable endoluminal prosthesis 1 according to the present invention deployed within the aorta, particularly from the aortic annulus **42** to the descending aorta and the arch which covers the coronaries **44** and the supra aortic branches **37**.

The implantable endoluminal prosthesis **1** according to the present invention comprises a self-expandable braided framework **20** able to expand from a radially compressed state in a delivery configuration to a radially expanded state and a radially collapsible valve body **10** made of an impermeable material, as shown FIGs. 8a to 8c.

The braided framework **20** has a proximal end **6** configured to extend toward the heart and a distal end **7** configured to extent toward away from the heart. The braided framework **20** comprises a main tubular body **3** comprising a lumen in a cylindrical form with a circular cross-section and a constant diameter at the distal end of the braided framework, a neck **5** comprising a lumen of cylindrical form with a circular cross-section and a constant diameter smaller than the one of said main tubular body **3** at the proximal end of the braided framework **20,** and a transition portion **4** extending between the proximal end of the main tubular body **3** and the distal end of the neck **5.** Said main tubular body **3,** said neck **5** and said transition portion **4** consist of an integrated continuous structure made of a multilayer braid and devoid of any impermeable cover layer. The radially collapsible valve body **10** is placed within the lumen of the neck **5.** In the fully expanded state, the total length of the main tubular body **3** and the transition portion **4** is at least 50 mm so that the wall of the braided framework **20** completely covers the aneurysm **40,** as shown in FIG.14.

The total length of the main tubular body **3** and the transition portion **4** is, preferably, at least 100 mm in fully expanded state in order to ensure fully covering aneurysmal portion of aorta with the self-expandable braided framework **20.** The total length is more preferably at least 150 mm, even more preferably at least 200 mm (still in fully expanded state as shown in FIG.8), so that the braided framework can have at least 20 mm of healthy landing zone in order to avoid endoleak, which is a main cause of recurrent aneurysms after implantation.

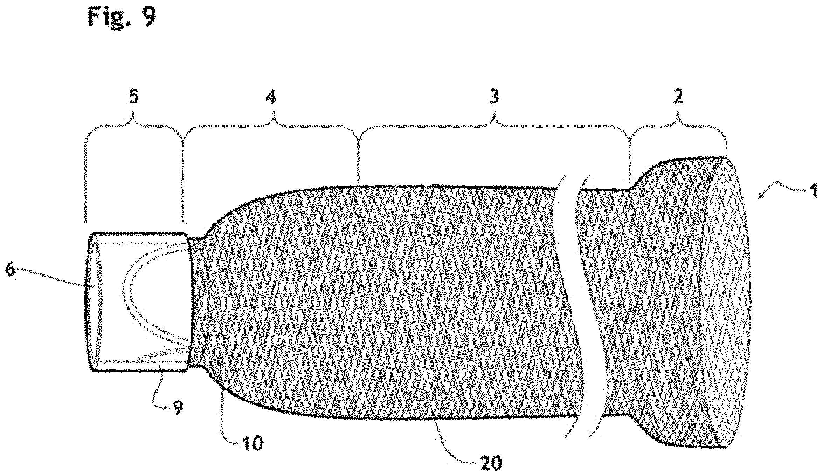

As a preferred embodiment, the self-expandable braided framework **20** further comprises an enlarged portion **2** between the distal end **7** of the braided framework **20** and the main tubular body **3** as illustrated in FIG.9. The diameter of the enlarged portion **2** increases toward the distal end **7** of the braided framework **20.** The enlarged portion **2** also reduce the risk of a device migration and endoleak after implantation.

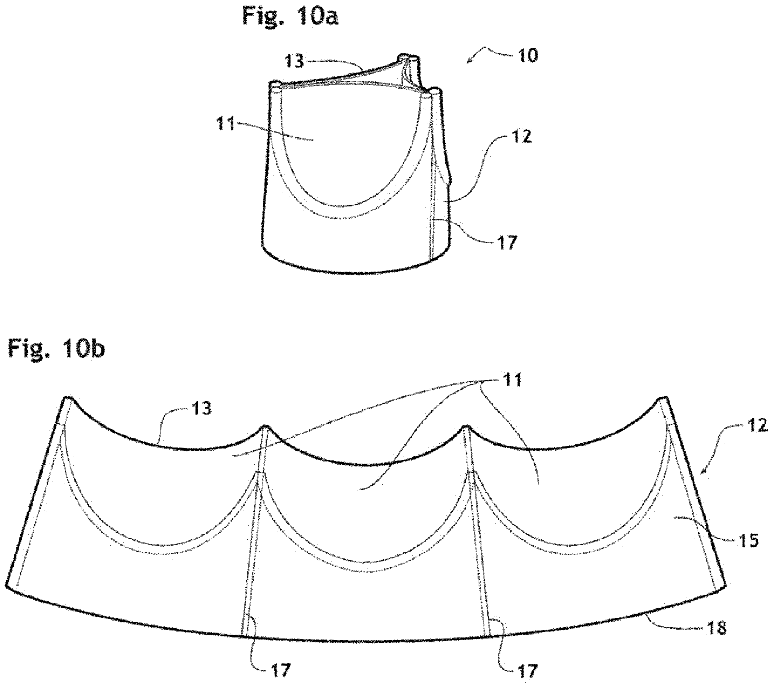

FIGs. 10a and 10b show in a more detailed manner the radially collapsible valve body **10** of the present invention. This valve body comprises a skirt **12** and leaflets **11** which are made of impermeable material. Said skirt **12** and leaflets **11** are preferably cut from a sheet of animal pericardial tissue, such as porcine pericardial tissue, or from another suitable synthetic or polymeric material. The pericardial tissue may be processed in accordance with tissue processing techniques that are per se known in the art of forming and treating tissue valve material. Leaflet **11** has a free edge **13** and a leaflet body **14.** Free edge **13** forms coaptation edge **13** of the finished valve body **10.** Leaflet body **14** is joined to a skirt **12.** Skirt **12** is preferably constructed from the same material as leaflets **11,** and comprises concaved portions **15,** reinforcing areas **17,** and a proximal portion **18.** Each concaved portion **15** is joined to a leaflet body **14** of a respective leaflet **11** by sutures or gluing. The valve body **10** is a truncated cone shape having an axis parallel to the one of the braided framework **20** and preferably comprises a reinforcing means, such as overlapped valve body material, metallic wire and plastic bar that are for example affixed to a wall of the skirt **12** between concaved portions **15** along the axis. This prevents the valve body **10** from turning inside out during the cardiac cycle and/or from migration of valve body placed in the braided framework. The proximal portion **18** of skirt **12** is preferably affixed to an inner wall of the proximal end **6** of the braided framework **20** with attaching means such as sutures and gluing.

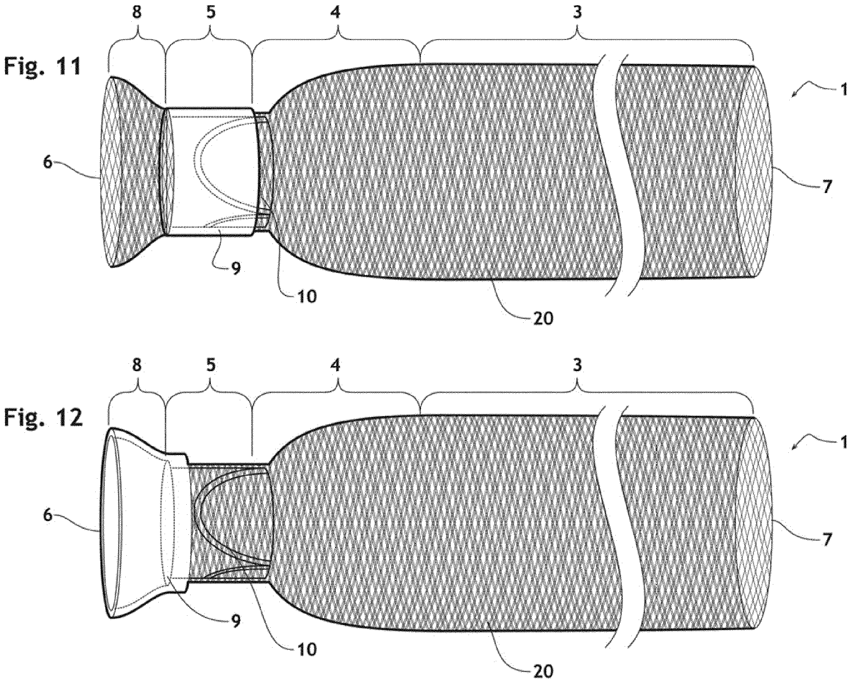

According to another embodiment, illustrated in FIGs.11 and 12 the self-expandable braided framework **20** further comprises a sealing portion **8** between the proximal end **6** of the braided framework **20** and the neck **5.** The diameter of the sealing portion **8** increases toward the proximal end **6** of the braided framework. The sealing portion **8** also reduces the risk of migration of the device away from the valve site after implantation.

In order to ensure sealing of the aneurysm **40** and prevent the blood flow from regurgitation, an impermeable biocompatible sleeve **9** can be used to clamp together both proximal ends, **18** and **6,** of skirt **12** and braided framework **20** and affixed by attaching means such as sutures and gluing as illustrated in FIG. 7. This also reduces the risk that wired edges of the proximal end **6** hurt the tissue of aortic annulus **42** when it is deployed in the body. Preferably, the impermeable biocompatible sheet **9** is elastic to accommodate to the change in the length and diameter of the braided framework between its delivery and deployed states.

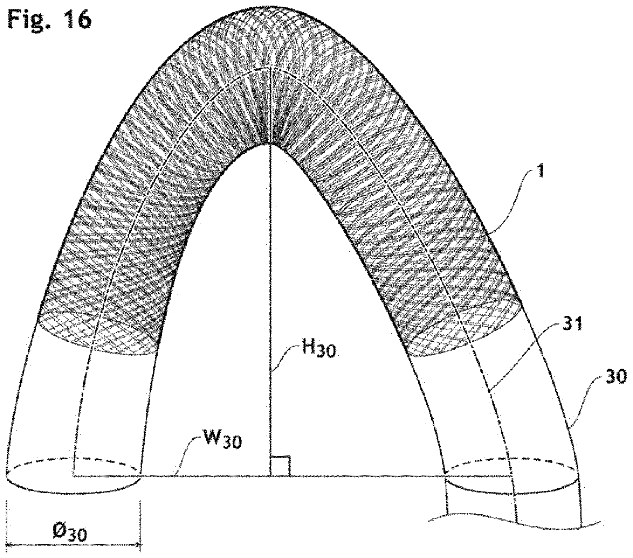

When the tubular body **2** is deployed in a curved lumen **30** as shown in FIG.16, its length ($L_{3\_dep}$) is measured along the midpoint **31** of the curve as indicated in FIG.18.

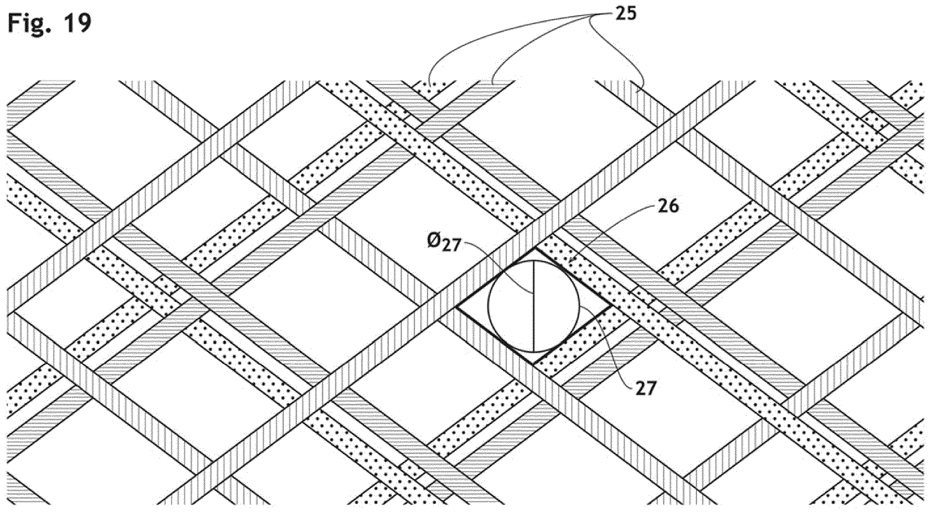

As depicted in FIG.19, the braided framework **20** comprises a plurality of layers **22, 23, 24** of wires **25** made of biocompatible material. The wires preferably have a diameter ($\varnothing_{25}$) of more than 180 $\mu$m, preferably at least 200 $\mu$m and at most 220 $\mu$m. Each layer of the braided framework **20** forms a mesh. When observed normal with respect to a wall, meshes of the braided frame **20** form a lattices with a plurality of level of wires **25.** Preferably, the meshes are interlocked with each other so as to form an interlocked multi-layer structure. The term "interlocked multi-layer" refers to a framework comprising multiple layers, **22, 23, 24,** whose plies are not distinct at the time of braiding, for example a given number of wires of the plies **22a** of the first layer **22** being interlocked with the plies **23a** of the second layer **23** and/or other layers **24.** Said interlocked multi-layer, for example, can be formed by using the braiding machine described in EP1248372. The braided framework **20** of the endoluminal prosthesis **1** is made of less than 150 wires **25,** preferably at least 90 wires at most 130 wires.

The surface coverage ratio (**SCR**) of the braided framework **20** is defined by the relation:

$$SCR = S_w/S_t$$

wherein: "$S_w$" is the actual surface covered by wires **25** composing the braided framework **20,** and "$S_t$" is the total surface area of the wall of the braided framework **20.** In a fully expanded state, **SCR** of the braided framework 20 is preferably at least 25% and at most 50%, preferably at least 30% and at most 40%, more preferably at most 35%.

The curve of the aortic arch **39** is generally defined by measuring the width ($W_{39}$) and height ($H_{39}$) of the curve as described by Ou et al. in J. Thrac. Cardiovasc. Surg. 2006; 132: 1105-1111. Width ($W_{39}$) is measured as the maximal horizontal distance between the midpoints **31** of the ascending and descending aorta **39** close to the axial plane going through the right pulmonary artery (RPA); and height ($H_{39}$) of the aortic arch is measured maximal vertical distance between ($W_{39}$) and the highest midpoint **31** of the aortic arch **39** as depicted in FIG. 16. The ratio $H_{39}/W_{39}$ is generally in a range of 0.5 to 0.9. For example, when the value is 0.9 (the worst scenario), the aortic arch is extremely acute as depicted in FIG. 16. This can cause a kinking of previously described "conventional" stents, which have poor hoop force. Furthermore, one will notice the difference of mesh opening between its straight form greater in comparison with the one deployed in a curve having about 0.6 of the H/W ratio (which is usually observed in healthy aortas). As one of the advantages of the present invention, even if the endoluminal prosthesis **1** is deployed in a C-curved lumen **30** with the $H_{30}/W_{30}$ ratio between 0.5 and 0.9, the braided framework **20** with a ratio $T_1/\varnothing_{25}$ of at least 3.5 (preferably 5.5, more preferably at least 6.5, even more preferably at least 7.5) , can provide a surface coverage ratio (**SCR**) within the desirable range along its outer curve **29,** i.e. at least 35%., resulting in maintaining the desired effects at the inlet of supra aortic branches **37** (i.e., laminar effect, improvement of perfusion) .

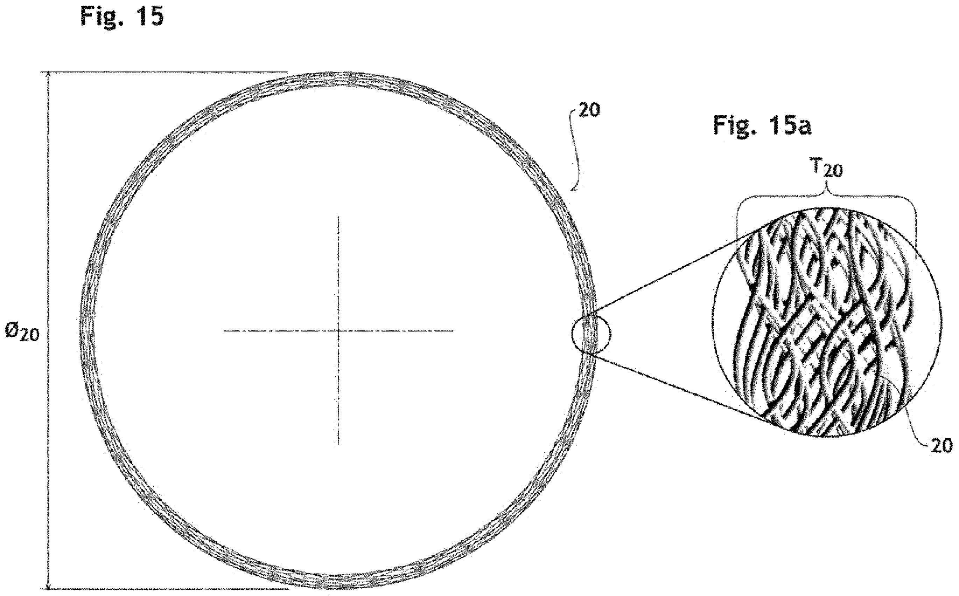

As another advantages of the present invention the braided framework **20,** having higher value of the ratio $T_{20}/\varnothing_{25}$, can effectively form a thrombus in the aneurysmal sac in comparison with a braided framework having lower $T_{20}/\varnothing_{25}$ ratio. The ratio ($T_{20}/\varnothing_{25}$) of the wall thickness ($T_{20}$) of the braided framework **20** to the wire diameter ($\varnothing_{25}$) being more than 2.0 characterizes the braided framework having more than a single layer of mesh. The greater the ratio $T_{20}/\varnothing_{25}$, the more layers the braided framework **20** will comprise. Each wire forming multiple-layers aligned substantially parallel in the wall, as shown in FIG. 15, works to make the blood flow be laminated which gets through the wall of the endoluminal prosthesis **1.**

Furthermore, interlocked multiple-layer configuration having a ratio $T_{20}/\varnothing_{25}$ higher than 3.5 brings about an important technical property: when it is deployed in a curved lumen having an H/W ratio between 0.5 and 0.9, the SCR can keep its desirable value, namely at least 25% and at most 50%, even at the outer side of the curve **29** as defined in FIGs 11 and 14. Since the mouths of the supra aortic branches are located at the outer side of the arch, it is most important to set an optimal opening size at the outer side when deployed in an aortic arch geometry in order to maintain desirable effects provided by the prosthesis. Wires of the interlocked multiple-layer configuration of the invention shift to keep a regular distance between adjacent parallel resulting in that the SCR can stays almost the same either in a curved state or in straight configuration. On the Contrary, when a conventional single-layer mesh-like tube having less than 2.0 of $T_{20}/\varnothing_{25}$ is deployed in a curved lumen, the SCR at the outer side of the curve are much lower than the SCR in a straight configuration. Therefore, the ratio $T_{20}/\varnothing_{25}$ of the braided framework **20** of the invention should be more than 2.0, preferably at least 3.5, more preferably at least 5.5, even more preferably at least 6.5, still even more preferably 7.5.

Studies and experiments carried by the inventor led to surprising and unexpected conclusions. The perfusion in the branches is improved in accordance with the increase of the ratio $T_{20}/\varnothing_{25}$. "Perfusion" is, in physiology, the process of a body delivering blood to capillary bed in its biological tissue. The terms "hypoperfusion" and "hyperperfusion" measure the perfusion level relative to a tissue's current need to meet its metabolic needs. Since the implantable medical device of the invention increases the perfusion in the supra aortic branches it covers, the functioning of the organs to which the supra aortic branches carries the blood is improved. Therefore, the ratio $T_{20}/\varnothing_{25}$ of the braided framework **20** of the invention should be more than 2.0, preferably at least 3.5, more preferably at least 5.5, even more preferably at least 6.5, still even more preferably 7.5.

As another surprising effect, against the expectation that a space between an aneurysmal wall and endoluminal prosthesis would be occluded by thrombus, the aneurysm including coronary arteries shrinks directly instead of forming thrombus in the aneurysmal sac while still maintaining the blood flow into the arteries. The inventor assumes that by sealing the beginning of aorta with its valve portion, undesired turbulence **53** are eliminated and desired smooth flow are created in this volume. It accelerates the non-turbulent blood flow entering the branches while decreasing the pressure under Venturi effect, resulting in shrinkage of the aneurysmal sac.

The biocompatible material used in the invention is preferably a metallic substrate selected from a group consisting of stainless steels (e.g., 316, 316L or 304); nickel-titanium alloys including shape memory or superelastic types (e.g., nitinol, Nitinol-DFT®-Platinum); cobalt-chrome alloys (e.g., elgiloy); cobalt-chromium-nickel alloys (e.g., phynox); alloys of cobalt, nickel, chromium and molybdenum (e.g., MP35N or MP20N); cobalt-chromium-vanadium alloys; cobalt-chromium-tungsten alloys; magnesium alloys; titanium alloys (e.g., TiC, TiN); tantalum alloys (e.g., TaC, TaN); L605;. Said metallic substrate is preferably selected from the group consisting of titanium, nickel-titanium alloys such as nitinol and Nitinol-DFT®-Platinum, any type of stainless steels, or a cobalt-chromium-nickel alloys such as Phynox®.

## Method of Deployment

According to one preferred method, the endoluminal prosthesis 1 of the invention is deployed by using an endoluminal prosthesis delivery apparatus. This apparatus is designed to be driven by an operator from the proximal site on through the vascular system so that the distal end of the apparatus can be brought close to the implantation site, where the prosthesis 1 can be unloaded from the distal end of the apparatus. The delivery apparatus comprises the prosthesis 1 itself, a prosthesis receiving region wherein the prosthesis has been introduced, a central inner shaft and a retracting sheath. Preferably, the apparatus further comprises a self-expanding holding means that is compressed within the sheath, the distal portion of which encircles the proximal potion of the prosthesis, and the proximal end of which is permanently joined to the inner shaft with a joint so as to provide the apparatus with a function of re-sheathing a partially unsheathed prosthesis into a retracting sheath. To deploy the prosthesis 1 at a desired location in the aorta, the distal end of the retracting sheath is brought to the aortic annulus and the retracting sheath is progressively withdrawn from over the prosthesis 1 toward the proximal end of the delivery apparatus. Once the sheath is adjacent the proximal end of the holding means, the prosthesis 1 is partially allowed to self-expand to a deployed shape. By continually retracting the sheath proximally, the holding means is released from the sheath and deploys while under the effect of the temperature of the organism and/or because of their inherent elasticity. In order to prevent a prosthesis migration after implantation, an oversized prosthesis 1 is generally chosen which has a diameter in its "nominal" expanded state being 10-40% greater than the diameter of the body lumen at the implantation site. Such prosthesis 1 exerts a sufficient radial force on an inner wall of the body lumen and is thus fixed firmly where it is implanted. Since, upon deployment, the radial force provided by the deployed part of the prosthesis 1 onto the wall of the aorta becomes greater than the grasping force of the deployed holding means in its deployed state, the holding means can release the prosthesis at the deployed position without undesired longitudinal displacement when retracting the inner shaft proximally together with the sheath.

## Claims

1. Implantable endoluminal prosthesis suitable for deployment from the aortic annulus to the aorta comprising:

   1) a self-expandable braided framework (**20**) extending along an axis able to expand from a radially compressed state in a delivery configuration to a radially expanded state, the self-expandable braided framework (**20**) being formed of braided wires having a given diameter ($\varnothing_{25}$) and having a proximal end (**6**) configured to extend toward the heart, and a distal end (**7**) configured to extent toward away from the heart, this self-expandable braided framework (**20**) comprising:

      a) toward the distal end (**7**), a main tubular body (**3**) comprising a lumen in a cylindrical form with a circular cross-section and a constant diameter;
      b) toward the proximal end (**6**), a neck (**5**) comprising a lumen in a cylindrical form with a circular cross-section and a constant diameter smaller than the one of said main tubular body (**3**); and
      c) a transition portion (**4**) extending between the proximal end (**6**) of the main tubular body (**3**) and the distal end of the neck (**5**), said main tubular body (**3**), said neck (**5**) and said transition portion (**4**) consisting of an integrated structure being devoid of any impermeable cover layer, and forming a wall having an average thickness ($T_{20}$),

   2) a radially collapsible valve body (**10**) comprising an impermeable material placed within the lumen of the neck (**5**),
   **characterized in that**, in radially expanded state, the total length of the main tubular body (**3**) and the transition portion (**4**) is at least 50 mm, the self-expandable braided framework (**20**) comprising plurality of wires (**25**) made of biocompatible material and forming a lattice when observed normal to a wall of the self-expandable braided framework (**20**), the lattice defining polygonal opening units (**26**), a ratio ($T_{20}/\varnothing_{25}$) of the average thickness ($T_{20}$) of a wall of the self-expandable braided framework (**20**) to the diameter ($\varnothing_{25}$) of wire (**25**) being greater than 2.0, the self-expandable braided framework (**20**) comprising less than 150 wires, preferably at least 90 wires and at most 130 wires.

2. Implantable endoluminal prosthesis according to claim 1, wherein the braided framework comprises plurality of layers (**22, 23, 24**) of the wires, each layer forming a mesh, the meshes are interlocked, the wires being integrated in the mesh of at least one of the adjacent layers.

3. Implantable endoluminal prosthesis according to either one of claim 1 or 2, wherein, in the radially expanded state, the total length of the main tubular body (**3**) and the transition portion (**4**) is at least 150 mm, preferably 200 mm.

4. Implantable endoluminal prosthesis according to any one of preceding claims, wherein the ratio ($T_{20}/\varnothing_{25}$) is at least 3.5, more preferably at least 5.5, even more preferably at least 6.5, still even more preferably at least 7.5.

5. Implantable endoluminal prosthesis according to any one of preceding claims, wherein the diameter of the wires (25) is larger than 180 μm, preferably at least 200 μm and at most 220 μm.

6. Implantable endoluminal prosthesis according to any one of preceding claims, wherein , in a fully expanded state, a surface coverage ratio (SCR) of said self-expandable braided framework (20) is at least 25% and at most 50%, preferably at least 30% and at most 40%, more preferably at most 35%.

7. Implantable endoluminal prosthesis according to any one of preceding claims, wherein the self-expandable braided framework (20) further comprises a sealing portion (8) between the proximal end (6) of the braided frame work and the neck (5), the diameter of the sealing portion (8) increasing toward the proximal end (6) of the braided framework.

8. Implantable endoluminal prosthesis according to any one of preceding claims, wherein the self-expandable braided framework (20) further comprises an enlarged portion (2) between the distal end (7) of the self-expandable braided framework (20) and the main tubular body (3), the diameter of this enlarged portion increasing toward the distal end (7) of the self-expandable braided framework (20).

9. Implantable endoluminal prosthesis according to any one of any one of preceding claims, wherein the biocompatible material is a metallic substrate selected from the group consisting of titanium, nickel-titanium alloys such as nitinol and Nitinol-DFT®-Platinum, any type of stainless steels, or a cobalt-chromium-nickel alloys such as Phynox®.

10. Implantable endoluminal prosthesis according to any one of preceding claims for use in treatment for cardiac valve dysfunction involving ascending aortic aneurysm.

11. Implantable endoluminal prosthesis for use according to claim 10 wherein the cardiac valve dysfunction is aortic valve regurgitation or aortic valve stenosis.

12. Implantable endoluminal prosthesis according to any one of claims 1 to 9 for use in improving perfusion of an organ by covering with said implantable endoluminal prosthesis orifices of the coronaries and the supra aortic branches which carries blood to the heart and the brain.

**Patentansprüche**

1. Implantierbare endoluminale Prothese, die zur Entfaltung vom Aortenannulus zur Aorta geeignet ist, umfassend:

1) einen selbstexpandierenden geflochtenen Rahmen (20), der sich entlang einer Achse erstreckt, die dazu in der Lage ist, von einem radial komprimierten Zustand in einer Abgabekonfiguration in einen radial expandierten Zustand zu expandieren, wobei der selbstexpandierende geflochtene Rahmen (20) aus geflochtenen Drähten gebildet ist, aufweisend einen bestimmten Durchmesser ($\varnothing_{25}$) und ein proximales Ende (6), das konfiguriert ist, um sich hin zum Herzen zu erstrecken, und ein distales Ende (7), das konfiguriert ist, um sich vom Herzen weg zu erstrecken, wobei dieser selbstexpandierende geflochtene Rahmen (20) Folgendes umfasst:

a) hin zum distalen Ende (7) einen rohrförmigen Hauptkörper (3), umfassend ein Lumen mit einer zylindrischen Form mit einem kreisförmigen Querschnitt und einem konstanten Durchmesser;
b) hin zum proximalen Ende (6) einen Hals (5), umfassend ein Lumen mit einer zylindrischen Form mit einem kreisförmigen Querschnitt und einem konstanten Durchmesser, der kleiner als derjenige des rohrförmigen Hauptkörpers (3) ist; und
c) einen Übergangsabschnitt (4), der sich zwischen dem proximalen Ende (6) des rohrförmigen Hauptkörpers (3) und dem distalen Ende des Halses (5) erstreckt, wobei der rohrförmige Hauptkörper (3), der Hals (5) und der Übergangsabschnitt (4) aus einer integrierten Struktur bestehen, die keine undurchlässige Deckschicht umfasst und eine Wand mit einer durchschnittlichen Dicke ($T_{20}$) aufweist,

2) einen radial klappbaren Ventilkörper (10), umfassend ein undurchlässiges Material, das innerhalb des Lumens des Halses (5) platziert ist,
**dadurch gekennzeichnet, dass**, im radial expandierten Zustand, die gesamte Länge des rohrförmigen Hauptkörpers (3) und des Übergangsabschnitts (4) mindestens 50 mm beträgt, wobei der selbstexpandierende geflochtene Rahmen (20) eine Vielzahl von Drähten (25) umfasst, die aus biokompatiblem Material hergestellt sind und ein Gitter bilden, wenn sie senkrecht zu einer Wand des selbstexpandierenden geflochtene Rahmens (20) betrachtet werden, wobei das Gitter polygonale Öffnungseinheiten (26) definiert, wobei ein Verhältnis ($T_{20}/\varnothing_{25}$) der durchschnittlichen Dicke ($T_{20}$) einer Wand des selbstexpandierenden geflochtenen Rahmens (20) zum Durchmesser ($\varnothing_{25}$) des Drahts (25) größer als 2,0 ist, wobei der selbstexpandierende geflochtene Rahmen (20) weniger als 150 Drähte umfasst, vorzugsweise weniger als 90 Drähte und höchstens 130 Drähte.

2. Implantierbare endoluminale Prothese nach Anspruch 1, wobei der geflochtene Rahmen eine Vielzahl von Schichten (22, 23, 24) der Drähte umfasst, wobei jede Schicht ein Netz bildet, die Netze verschränkt sind, wobei die Drähte in das Netz von mindestens einer der benachbarten Schichten integriert sind.

3. Implantierbare endoluminale Prothese nach ein einem der Ansprüche 1 oder 2, wobei im radial expandierten Zustand die gesamte Länge des rohrförmigen Hauptkörpers (3) und des Übergangsabschnitts (4) mindestens 150 mm, bevorzugt 200 mm beträgt.

4. Implantierbare endoluminale Prothese nach ein einem der vorhergehenden Ansprüche, wobei das Verhältnis ($T_{20}/\varnothing_{25}$) mindestens 3,5 ist, insbesondere mindestens 5,5, noch bevorzugter mindestens 6,5 und noch bevorzugter mindestens 7,5.

5. Implantierbare endoluminale Prothese nach einem der vorhergehenden Ansprüche, wobei der Durchmesser der Drähte (25) größer als 180 $\mu$m, vorzugsweise mindestens 200 $\mu$m und höchstens 220 $\mu$m ist.

6. Implantierbare endoluminale Prothese nach einem der vorhergehenden Ansprüche, wobei in einem vollständig implantierten Zustand ein Flächenabdeckungsverhältnis (SCR) des selbstexpandierenden geflochtenen Rahmens (20) mindestens 25 % und höchstens 50 %, vorzugsweise mindestens 30 % und höchstens 40 %, insbesondere höchstens 35 % ist.

7. Implantierbare endoluminale Prothese nach einem der vorhergehenden Ansprüche, wobei der selbstexpandierende geflochtene Rahmen (20) weiter einen Dichtungsabschnitt (8) zwischen dem proximalen Ende (6) des geflochtenen Rahmens und dem Hals (5) aufweist, wobei sich der Durchmesser des Dichtungsabschnitts (8) hin zum proximalen Ende (6) des geflochtenen Rahmens erhöht.

8. Implantierbare endoluminale Prothese nach einem der vorhergehenden Ansprüche, wobei der selbstexpandierende geflochtene Rahmen (20) weiter einen erweiterten Abschnitt (2) zwischen dem distalen Ende (7) des selbstexpandierenden geflochtenen Rahmens (20) und dem rohrförmigen Hauptkörper (3) umfasst, wobei sich der Durchmesser dieses erweiterten Abschnitts hin zum distalen Ende (7) des selbstexpandierenden geflochtenen Rahmens (20) erhöht.

9. Implantierbare endoluminale Prothese nach einem der vorhergehenden Ansprüche, wobei das biokompatible Material ein metallisches Substrat ist, ausgewählt aus der Gruppe, bestehend aus Titan, Nickel-Titan-Legierungen wie z. B. Nitinol und Nitinol-DM-Platin, jeder Art von Edelstahl oder Kobalt-Chrom-Nickel-Legierungen wie z. B. Phynox®.

10. Implantierbare endoluminale Prothese nach einem der vorhergehenden Ansprüche zur Verwendung bei der Behandlung von Herzklappen-Funktionsstörungen, die ein aufsteigendes Aortaaneurisma einschließen.

11. Implantierbare endoluminale Prothese zur Verwendung nach Anspruch 10, wobei die Herzklappen-Funktionsstörung eine Aortenklappeninsuffizienz oder Aortenklappenstenose ist.

12. Implantierbare endoluminale Prothese nach einem der Ansprüche 1 bis 9 zur Verwendung bei der Verbesserung der Perfusion eines Organs durch Abdecken mit der implantierbaren endoluminalen Prothese von Öffnungen der Koronargefäße und der supraaortalen Zweige, die Blut zum Herzen und zum Gehirn transportieren.

**Revendications**

1. Prothèse endoluminale implantable appropriée pour le déploiement de l'anneau aortique à l'aorte comprenant :

    1) une structure tressée auto-expansible (20) s'étendant le long d'un axe pouvant subir une expansion d'un état radialement comprimé dans une configuration de pose à un état radialement expansé, la structure tressée auto-expansible (20) étant formée avec des fils tressés ayant un diamètre donné ($\varnothing_{25}$) et ayant une extrémité proximale (6) configurée pour s'étendre vers le cœur, et une extrémité distale (7) configurée pour s'étendre vers, à distance du cœur, cette structure tressée auto-expansible (20) comprenant :

        a) vers l'extrémité distale (7), un corps tubulaire principal (3) comprenant une lumière se présentant sous une forme cylindrique avec une section transversale circulaire et un diamètre constant ;

b) vers l'extrémité proximale (6), un col (5) comprenant une lumière se présentant sous une forme cylindrique avec une section transversale circulaire et un diamètre constant inférieur à celui dudit corps tubulaire principal (3) ; et

c) une partie de transition (4) s'étendant entre l'extrémité proximale (6) du corps tubulaire principal (3) et l'extrémité distale du col (5),

ledit corps tubulaire principal (3), ledit col (5) et ladite partie de transition (4) se composant d'une structure intégrée qui est dépourvue de couche de couverture imperméable, et formant une paroi ayant une épaisseur moyenne ($T_{20}$),

2) un corps de valve radialement pliable (10) comprenant un matériau imperméable placé dans la lumière du col (5), **caractérisée en ce que**, à l'état radialement expansé, la longueur totale du corps tubulaire principal (3) et de la partie de transition (4) est d'au moins 50 mm, la structure tressée auto-expansible (20) comprenant une pluralité de fils (25) réalisés avec un matériau biocompatible et formant un treillis lorsqu'ils sont observés perpendiculairement à une paroi de la structure tressée auto-expansible (20), le treillis définissant des unités d'ouverture polygonales (26), un rapport ($T_{20}/\varnothing_{25}$) de l'épaisseur moyenne ($T_{20}$) d'une paroi de la structure tressée auto-expansible (20) sur le diamètre ($\varnothing_{25}$) du fil (25) étant supérieur à 2,0, la structure tressée auto-expansible (20) comprenant moins de 150 fils, de préférence au moins 90 fils et au maximum 130 fils.

2. Prothèse endoluminale implantable selon la revendication 1, dans laquelle la structure tressée comprend une pluralité de couches (22, 23, 24) de fils, chaque couche formant une maille, les mailles sont entrelacées, les fils étant intégrés dans la maille d'au moins l'une des couches adjacentes.

3. Prothèse endoluminale implantable selon l'une des revendications 1 ou 2, dans laquelle, à l'état radialement expansé, la longueur totale du corps tubulaire principal (3) et de la partie de transition (4) est d'au moins 150 mm, de préférence de 200 mm.

4. Prothèse endoluminale implantable selon l'une quelconque des revendications précédentes, dans laquelle le rapport ($T_{20}/\varnothing_{25}$) est d'au moins 3,5, encore de préférence d'au moins 5,5, encore de préférence d'au moins 6,5, de manière préférée entre toutes d'au moins 7,5.

5. Prothèse endoluminale implantable selon l'une quelconque des revendications précédentes, dans laquelle le diamètre des fils (25) est supérieur à 180 $\mu$m, de préférence d'au moins 200 $\mu$m et au maximum de 220 $\mu$m.

6. Prothèse endoluminale implantable selon l'une quelconque des revendications précédentes, dans laquelle, dans un état complètement expansé, un rapport de couverture de surface (SCR) de ladite structure tressée auto-expansible (20) est d'au moins 25%, et au maximum de 50%, de préférence d'au moins 30% et au maximum 40%, encore de préférence au maximum de 35%.

7. Prothèse endoluminale implantable selon l'une quelconque des revendications précédentes, dans laquelle la structure tressée auto-expansible (20) comprend en outre une partie d'étanchéité (8) entre l'extrémité proximale (6) de la structure tressée et le col (5), le diamètre de la partie d'étanchéité (8) augmentant vers l'extrémité proximale (6) de la structure tressée.

8. Prothèse endoluminale implantable selon l'une quelconque des revendications précédentes, dans laquelle la structure tressée auto-expansible (20) comprend en outre une partie agrandie (2) entre l'extrémité distale (7) de la structure tressée auto-expansible (20) et le corps tubulaire principal (3), le diamètre de cette partie agrandie augmentant vers l'extrémité distale (7) de la structure tressée auto-expansible (20) .

9. Prothèse endoluminale implantable selon l'une quelconque des revendications précédentes, dans laquelle le matériau biocompatible est un substrat métallique choisi dans le groupe comprenant le titane, des alliages de nickel-titane tel que le nitinol et le nitinol-DFT®-platine, n'importe quel type d'aciers inoxydables ou des alliages de cobalt-chrome-nickel tel que le Phynox®

10. Prothèse endoluminale implantable selon l'une quelconque des revendications précédentes, prévue pour être utilisée pour le traitement d'une dysfonction de la valvule cardiaque impliquant un anévrisme aortique ascendant.

11. Prothèse endoluminale implantable destinée à être utilisée selon la revendication 10, dans laquelle la dysfonction de la valvule cardiaque est une régurgitation de la valvule aortique ou une sténose de la valvule aortique.

12. Prothèse endoluminale implantable selon l'une quelconque des revendications 1 à 9, destinée à être utilisée pour améliorer la perfusion d'un organe en recouvrant avec ladite prothèse endoluminale implantable, les orifices des artères coronaires et les branches supra-aortiques qui amènent le sang au coeur et au cerveau.

Fig. 1
(Prior Art)

Fig. 2
(Prior Art)

Fig. 3
(Prior Art)

Fig. 4
(Prior Art)

Fig. 5
(Prior Art)

EP 3 402 446 B1

Fig. 6
(Prior Art)

Fig. 7

Fig. 8a

Fig. 8b

Fig. 8c

Fig. 9

Fig. 10a

Fig. 10b

Fig. 11

Fig. 12

EP 3 402 446 B1

Fig. 13

Fig. 14

Fig. 15a

Fig. 15

**Fig. 16**

$H_{30}$

$W_{30}$

$\varnothing_{30}$

1

31

30

EP 3 402 446 B1

Fig. 18

$L_{3\_dep}$

1

Fig. 17

1

$L_{3\_exp}$

$\varnothing_{3\_exp}$

Fig. 19

**EP 3 402 446 B1**

**REFERENCES CITED IN THE DESCRIPTION**

**Patent documents cited in the description**

- US 7588597 B
- US 8192484 B
- US 2013144373 A
- EP 1248372 A

**Non-patent literature cited in the description**

- **OU et al.** *J. Thrac. Cardiovasc. Surg.,* 2006, vol. 132, 1105-1111